(19) Europäisches Patentamt
European Patent Office
Office européen des brevets

(11) Numéro de publication: **0 159 293**
**A1**

(12) **DEMANDE DE BREVET EUROPEEN**

(21) Numéro de dépôt: 85810146.2

(22) Date de dépôt: 01.04.85

(51) Int. Cl.4: A 61 L 15/06
C 08 G 69/10

(30) Priorité: 02.04.84 CH 1659/84

(43) Date de publication de la demande: 23.10.85 Bulletin 85/43

(84) Etats contractants désignés: AT BE CH DE FR GB IT LI LU NL SE

(71) Demandeur: BATTELLE MEMORIAL INSTITUTE
7 route de Drize
CH-1227 Carouge/Genève(CH)

(72) Inventeur: Bichon, Daniel
16, rue de Vallard
F-74240 Gaillard(FR)

(72) Inventeur: Borloz, William
16, rue de la Morâche
CH-1260 Nyon(CH)

(74) Mandataire: Dousse, Blasco et al,
7, route de Drize
CH-1227 Carouge/Genève(CH)

(54) **Matière polymérique filmogène utilisable comme pansement et sa fabrication.**

(57) On dépose sur la peau une solution dans un solvant hydrosoluble d'un polymère de formule

$$-(NH-CH[(CH_2)_n-COO-CR^1R^2-OOC-R]-CO)_x-$$

où $R^1$ et $R^2$ sont des alcoyles ou l'hydrogène et R est aliphatique ou aromatique, de manière à former une couche qu'on lave ensuite à l'eau pour éliminer le solvant et réaliser ainsi un revêtement filmogène protecteur sur la peau.

EP 0 159 293 A1

0159293

# MATIERE POLYMERIQUE FILMOGENE UTILISABLE COMME PANSEMENT ET SA FABRICATION

La présente invention a pour objet une matière polymérique filmogène en solution utilisable comme pansement et, notamment, en tant que remplaçant provisoire de tissus cutanés lésés ou détruits, par exemple dans le traitement des brûlures, blessures étendues et autres affections semblables. L'invention a également pour objet la préparation de films de ladite matière.

On trouve sur le marché et dans l'état de la technique un grand nombre de produits filmogènes destinés à protéger les blessures et plus particulièrement les brûlures où l'épiderme ou le derme a été détruit sur une grande surface.

Ainsi, beaucoup de pansements absorbables (ou biodégradables) sont basés sur des polymères naturels: collagène, fibrine ou complexes de collagène avec polysaccharides, par exemple EP-A-92200, EP-A-91821, EP-A-90997; d'autres pansements proviennent de polymères dérivés de la kératine hydrolysée (EP-A-89152) ou de la cellulose oxydée (CS-A-8107668) ou de polymères synthétiques comme dans le document EP-A-86613. La plupart de ces textes décrivent des procédés de fabrication de pansements sous forme de membranes semi-perméables, ou microporeuses comportant en général 2 couches. Il est en effet connu que pour qu'une "peau artificielle" soit efficace, elle doit avoir une structure plus ou moins poreuse pour permettre la régénération des tissus (voir, par exemple, Milos Chvapil: Considerations ou Manufacturing Principles of a Synthetic Burn Dressing: Journal of Biomedical Materials Research <u>16</u>, 245 (1982)).

Selon le type de blessures (brûlures infectées, escarres, ulcères variqueux, etc..), il peut être nécessaire de disposer de pansements absorbant fortement l'humidité pour éviter la macération des tissus ou, au contraire, de pansements à membrane semi-perméable qui, tout en ayant une certaine perméabilité à la vapeur d'eau, empêchent qu'une trop grande partie des liquides physiologiques diffusent vers l'extérieur. Les propriétés que doivent donc posséder de telles matières filmogènes apparaîssent donc, dans certains cas, comme contradictoires.

Le mode d'application de la matière de pansement est également important: Ainsi, s'il est facile d'appliquer des pansements préfabriqués sur les parties planes du corps (dos, cuisses, bras), d'autres zones de surface irrégulières comme les mains, le visage et les articulations se prêtent mal à ce type d'application. Aussi, on a imaginé de former le pansement in situ par nébulisation sur la plaie d'une solution d'un polymère filmogène. Toutefois, pour réaliser de telles solutions, on est obligé d'employer des solvants organiques qui présentent de gros inconvénients dans le cas de blessures importantes, ceux-ci étant toxiques et leur application douloureuse (voir DE-A-3133896, CH-A-530795 et US-A-3928556)

Il est également possible pour pallier les inconvénients ci-dessus, d'appliquer une solution aqueuse de polymère sur la blessure, mais ce procédé présente deux défauts, à savoir le temps nécessaire pour évaporer le solvant de manière que le film se forme et, plus grave encore, le fait que le polymère est soluble dans l'eau ce qui interdit tout contact ultérieur de la région blessée avec celle-ci. Une solution au problème ci-dessus a été décrite par P. NATHAN et al. Trans. Amer. Soc. Artif. Int. Organs 22, 30 (1976). On forme le pansement en deux temps: on étale d'abord sur la plaie une couche de polyéthylène glycol 400 ou PEG 400 (un oligomère biocompatible jouant le rôle de solvant) et on saupoudre cette couche avec de fines particules de polyméthacrylate d'hydroxyéthyle (HEMA) non réticulé, soluble dans le PEG 400. On obtient de la sorte une solution de poly (HEMA) à 50% environ dans le PEG, qui se présente sous forme d'un film sec et élastique. Ce même type de formation d'un pansement est décrit dans les documents DE-A-2933250 et FR-A-2433561 (KURARAY).

Cependant, cette technique présente certains inconvénients, à savoir:

- le polymère n'est pas insoluble dans l'eau, ce qui empêche les lavages et les traitements d'hydrothérapie;
- il est difficile, lors du saupoudrage, d'obtenir un film homogène d'épaisseur constante suffisamment souple pour se plier aux mouvements des articulations et de la peau;
- étant donné le manque de biodégradabilité du polymère utilisé, des fragments de celui-ci peuvent rester captifs dans les tissus lors

de leur cicatrisation.

La présente invention remédie à ces inconvénients et propose une matière filmogène en solution donnant, après élimination de solvant, un film souple pour pansements dont l'épaisseur peut être rendue pratiquement uniforme, qui est insoluble dans l'eau et, de plus, dans certaines de ses formes, entièrement biodégradable et absorbable par les tissus du corps. Les films issus de l'invention sont, dans certaines formes d'exécution, parfaitement adhérents à la plaie et se prêtent, sans se déformer, se déchirer et se décoller, aux mouvements et flexions de l'organe traité. Par ailleurs, ils favorisent la cicatrisation.

Enfin, la matière issue de l'invention est, le plus souvent, soluble dans la plupart des solvants inoffensifs courants convenant aux médicaments (en effet, les polymères faisant partie de la technique ne sont, en général, solubles que dans certains solvants spéciaux (DMF, pyridine, $F_3CCOOH$) dont l'emploi est incommode pour les produits pharmaceutiques). Elle est de plus thermoformable ce qui le distingue notamment des polypeptides de synthèse actuellement connus qui ne sont généralement pas miscibles avec les plastifiants biocompatibles usuels (polyalcoylène glycols) et, de ce fait, ne sont pas thermoplastiques.

La présente matière est constituée de films d'un polymère polypeptidique estérifié de formule:

$$\begin{array}{l} -(NH-\underset{|}{C}H-CO)_x- \\ \quad\quad\;\; (CH_2)_n-COO-CR^1R^2-OOC-R \end{array} \qquad (I)$$

dans laquelle $R^1$ et $R^2$ sont des groupes alcoyles ou l'hydrogène avec R un reste aliphatique ou aromatique substitué ou non, ou bien $R_2$ est l'hydrogène ou un alcoyle et $R^1$ et R liés l'un à l'autre sont des groupes méthylène ou méthényle substitués ou non, n vaut 1 ou 2 et x est tel que la masse moléculaire soit d'au moins 5000 D.

Comme on le voit de par la formule I, le polymère permettant de réaliser les présents films est un polyaspartate ou polyglutamate estérifié par un dérivé d'acyloxyméthanol ($HO-CR^1R^2-OOCR$) dans lequel R est, soit un reste organique quelconque, soit relié à $R^1$

de manière à former un cycle. Par "quelconque" on veut dire que la nature et la structure du groupe R n'est pas critique et que, pour l'instant, on n'a pas rencontré de cas où, R faisant partie de composés courants à fonction RCOO-, le composé correspondant de l'invention ne puisse être obtenu. On préfère, cependant, utiliser des composés dans lequel R est un groupe aliphatique ou aromatique substitué ou non substitué, les substituants étant choisis parmi les fonctions organiques biocompatibles. Parmi les groupes R préférés, on peut citer les groupes méthyle, éthyle, propyle, isopropyle, isobutyle, tert-butyle, phényle, benzyle et autres groupes similaires. D'autres composés sont naturellement possibles, mais il est évident que pendant le temps limité dont a disposé l'inventeur, il n'a pu les envisager tous.

Lorsque R et $R^1$ sont reliés ensemble de façon à réaliser une liaison carbone-carbone saturée ou non, ces atomes de carbone peuvent être, ou non, substitués par des restes aliphatiques ou aromatiques. On donne ci-dessous quelques exemples non limitatifs de tels groupes ester-lactal

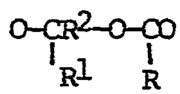

substitués ou non correspondant à la définition susmentionnée: groupe diméthylène $-CH_2-CH_2-$; groupe diméthyléthylène $-CH(CH_3)-CH(CH_3)-$; groupe vinylène $-CH=CH-$; groupe 1,2-phénylène 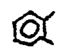 ; cyclohexénylène 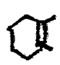 ; groupe cyclopenténylène 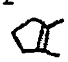 ; cyclopentadiénylène 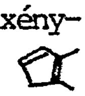 et autres.

Le polymère peut également se présenter sous la forme de copolymère avec d'autres polyaminoacides. Dans ce cas, on aura un copolymère de formule:

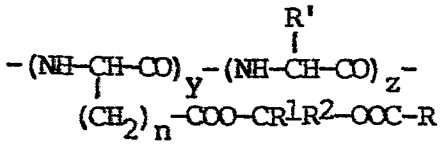 (II)

où R est un reste d'acide aminé estérifié ou non quelconque, les groupes R' des unités $-(NH-CHR'-CO)$ pouvant être identiques ou

différents dans la chaîne du copolymère, avec $y + z = x$, la valeur de x étant toujours choisie pour que le copolymère ait une masse moléculaire moyenne d'au moins 5000 D. Bien entendu, si R' est identique au groupe $-(CH_2)_n-COO-CR^1R^2-OOC-R$, les n étant cependant différents (l'un d'entre eux valant 1 et l'autre 2), on aura un copolymère estérifié d'acide glutamique et aspartique. Cependant, en règle générale, on préfère pour R' avoir des groupements différents, tels que, par exemple méthyle (alanine), isopropyle (valine), isobutyle (leucine et isoleucine), benzyle (phénylalanine), etc.. En principe, tous les autres acides aminés sont également possibles quoique, pour des raisons évidentes, on n'ait pu les essayer tous. R' peut également désigner un reste d'acide glutamique ou aspartique non estérifié, ou estérifié partiellement par un alcool quelconque, par exemple MeOH ou EtOH, c'est-à-dire, par exemple, $-(CH_2)_n-COOH$ ou $-(CH_2)_n-COOMe$.

On pourra également avoir, indifféremment, des acides aminés de la série L ou D. Les acides aminés de la série L (ou naturels) sont les plus intéressants car les polypeptides les contenant sont dégradables par les enzymes (protéases) du corps humain, alors que les polypeptides constitués d'unités D ne le sont pas. On peut mettre à profit cette différence grâce à des copolymères comprenant des aminoacides D et L, ceci afin de disposer de polymères dont la vitesse de dégradation est modifiée.

Revenant à des considérations plus générales, il faut noter que la proportion molaire, dans le copolymère II, de l'autre polyaminoacide libre ou partiellement estérifié permet également dans une notable mesure de régler la vitesse de biodégradation du copolymère en fonction des agents présents dans l'organisme au site de destination du mélange de copolymère et du médicament à administrer, (c'est-à-dire dans l'organe où le médicament doit agir). Ainsi, par exemple, si le copolymère est un copolymère de polyglutamate I et de leucine, on choisira la préparation molaire relative des deux constituants en fonction de la vitesse relative de dégradation, au lieu considéré, du polyglutamate et de la polyleucine. En règle générale, le rapport z/y peut varier de 1 à 30, mais ces limites peuvent être dépassées si besoin est. Bien entendu, dans le cas où le groupe R' ne désigne pas un groupe de nature unique dans la chaîne du

copolymère, c'est-à-dire, par exemple, lorsque l'un des R' désigne un reste d'acide aminé libre et qu'un autre R' désigne un reste d'amino-acide estérifié, on pourra, pour plus de commodité désigner les variantes de R' par les signes R", R''', etc.. La formule générale d'un tel copolymère peut alors être schématisée comme suit:

$$-(NH-\underset{(CH_2)_n-COO-CR^1R^2-OOCR}{\underset{|}{CH}}-CO)_y-(NH-\overset{R'}{\overset{|}{CH}}-CO)_z-(NH-\overset{R''}{\overset{|}{CH}}-CO)_u-(NH-\overset{R'''}{\overset{|}{CH}}-CO)_v$$

où la somme des y, z, u, v,..., etc est égale à x; u, v, etc.. pouvant être bien entendu nuls si le reste désigné par R' est de nature unique. Un cas typique où le copolymère présente des R' et R" distincts est celui où ces groupes désignent des restes d'acide glutamique et/ou aspartique estérifiés et non estérifiés, la formule schématique d'un tel polymère (dans le cas d'espèce, partiellement méthylé) se présentant comme suit:

$$-(NH-\underset{(CH_2)_n-COO-CR^1R^2-OOCR}{\underset{|}{CH}}-CO)_y-(NH-\overset{(CH_2)_n-COOH}{\overset{|}{CH}}-CO)_z-(NH-\overset{(CH_2)_n-COOMe}{\overset{|}{CH}}-CO)_u-$$

La nature du groupe R peut également influencer la vitesse de dégradation du polymère I. Ainsi, par exemple, si R est un groupe volumineux ou encombré (par exemple tert-butyle), la dégradation sera plus lente qu'avec un groupe méthyle ou éthyle.

Il est bien entendu que, du point de vue isomérie optique, les polymères de l'invention peuvent comprendre des éléments de configuration L ou D ou des mélanges racémiques ou, encore, des polymères où une des configurations domine. Les propriétés biochimiques de ces divers assemblages ne sont, bien évidemment, pas identiques, les polymères où dominent les formes naturelles (L) étant plus accessibles à la dégradation enzymatique. On peut donc en contrôler la dégradabilité en dosant, dans le copolymère les proportions relatives de l'une à l'autre forme.

Les polymères I et copolymère II sont insolubles dans l'eau et généralement solubles dans un ou plusieurs des solvants usuels tels

que l'acétone, la méthyléthyl cétone (MEK), le tetrahydrofurane (THF), le dioxanne, l'acétate d'éthyle, le monoglyme, le diglyme et autres, ce qui permet leur transformation aisée en la matière filmogène de l'invention. Les polymères I et II suivant leur structure peuvent être insolubles ou solubles dans les solvants chlorés, par exemple le chloroforme. Dans certains cas d'insolubilité dans le chloroforme, on peut y remédier si on ajoute à un tel solvant un peu d'acétone. Cette faculté de dissolution dans de nombreux solvants miscibles ou non miscibles à l'eau les rend également compatibles sans autre avec de nombreux médicaments liquides ou solubles dans les mêmes solvants.

Par ailleurs, suivant sa structure, le polymère I est très souvent parfaitement compatible avec les polyalcoylène glycols (polyéthylène glycol et polypropylène glycol), ce qui permet d'utiliser ces polyéther glycols comme plastifiants du polymère I et de fournir ainsi un mélange homogène de bas point de fusion. On peut facilement incorporer toute une gamme de médicaments thermolabiles à un tel mélange (fusion à des températures de l'ordre de 40 à 60°C) lors de la formation des films. De plus, la présence de polyalcoylène glycols très hydrophiles permet d'augmenter la susceptibilité du polymère et du copolymère aux liquides aqueux biologiques et faciliter leur dégradation enzymatique in situ. A titre comparatif, il est à remarquer que les polypeptides de synthèse connus ne possèdent pas ces propriétés favorables de solubilité et de compatibilité avec les PEG. Ainsi, par exemple, pour former des films d'acide polyglutamique présentant une résistance mécanique appréciable et une certaine insolubilité dans l'eau, on doit utiliser des solutions dans des solvants relativement malaisés à manipuler et peu appréciés en pharmacie tels que diméthyl formamide (DMF) et acides dichloracétique (DCA) et trifluoroacétique (TFA). Les films d'acide polyglutamique obtenus à partir de solutions aqueuses, (à pH 7,4, c'est-à-dire où l'acide est au moins en partie sous forme salifiée) n'ont aucune résistance mécanique et sont rapidement dissous dans l'eau, ce qui rend un tel polymère entièrement impropre à la fabrication de matières filmogènes telle que celle de l'invention. Il en est d'ailleurs de même des mélanges acide polyglutamique-polyethylène glycol qui sont instantanément solubles dans l'eau.

La biodégradation du polymère I peut être schématisée comme suit:

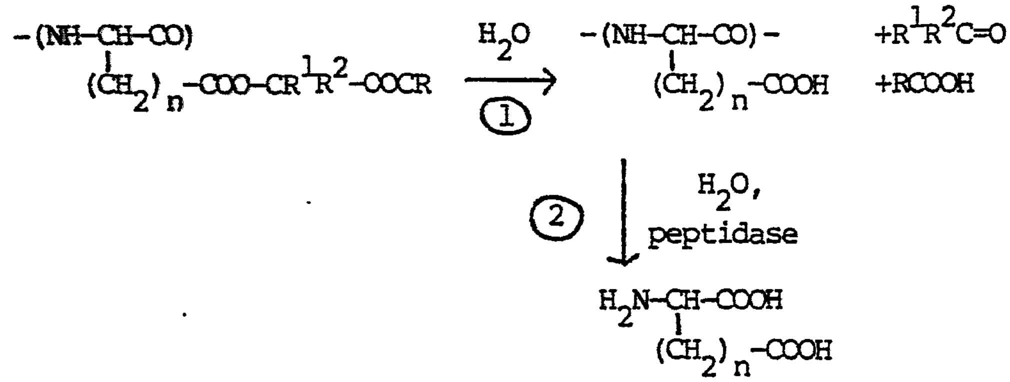


La réaction (2) est consécutive à la réaction (1) et, de ce fait, la biodégradation du polymère sera d'autant plus rapide que la vitesse d'hydrolyse de l'ester lactal ou acyloxyalcoylique est plus grande. Le mécanisme d'hydrolyse de ce type d'esters est connu en soi et a été mis en évidence lors de l'étude d'esters de l'ampicilline (par exemple la pivampicilline), voir à ce sujet la référence suivante: "Design of biopharmaceutical Properties through Prodrugs and Analogs", B. Roche, éditeur, American Pharcaceutical Association (ISBN 0-917330-16-1), pages 212 et 354 (1977). Il est à noter que lorsque $R^2$ est l'hydrogène et $R^1$ un alcoyle (par exemple méthyle), le composé résultant de la réaction 1 est un aldéhyde $R^1$-CHO (par exemple l'acétaldéhyde), de tels aldéhydes étant biologiquement plus avantageux que le métaldéhyde obtenu lorsque $R^1 = R^2 = H$ pour des raisons de toxicité. En ce qui concerne cet aspect de l'invention, les produits résultant de la réaction 1 lorsque R et $R^1$ sont reliés l'un à l'autre (des céto-acides ou acide-aldéhydes) sont également très avantageux en raison de leur toxicité négligeable. Ainsi, si l'association R-$R^1$ correspond aux groupes éthylène ou 1,2-phénylène, les produits de dégradation seront, respectivement, les acides 3-formyl-propionique et O-formyl-benzoïque lentement éliminés par l'organisme sans réactions secondaires.

On peut préparer le polymère I par estérification directe d'un sel du polyaminoacide correspondant avec un halogénure d'acyloxyméthyle (X-$CR^1R^2$-OCO-R (III)) ou X peut être le chlore, le brome ou l'iode. Le sel de polyaminoacide est, de préférence, un sel d'amine tertiaire (par exemple de tributylamine ou de triéthylamine). Une telle technique est connue en soi de par W.V. DACHNE; J. Med. chem.

13, 607 - 12, 1971). Par ailleurs, la synthèse des composés III (X = Cl) est connue (voir Z.H. ULICH, J.A.C.S. 43, 662 (1921)) et consiste à faire réagir le chlorure d'acide RCOCl avec le formaldéhyde en présence d'une quantité catalytique de $ZnCl_2$ anhydre.

Le polyaminoacide ou co-polyaminoacide dont l'estérification fournit le polymère I ou le copolymère II s'obtient facilement par les moyens habituels comprenant l'estérification par un alcool inférieur du carboxyle latéral d'un acide de formule

$$\begin{array}{l} H_2N-CH-COOH \\ \quad\quad\; | \\ \quad\quad (CH_2)_n-COOH, \end{array}$$

la transformation de l'ester en N-carboxyanhydride correspondant (NCA) par le phosgène en milieu dioxane ou THF, la polymérisation du NCA en polyaminoacide estérifié et l'hydrolyse du groupe ester protecteur en milieu alcalin ou par l'acide trifluoroacétique. De telles méthodes sont connues en soi (voir par exemple Encyclopedia of Polymer Science and Technology; N-carboxyanhydrides, vol II, page 837). Lorsqu'on désire parvenir à un copolymère ou R' désigne un carboxyle latéral partiellement estérifié (R' = $-(CH_2)_n-COOH$ et R" = $-(CH_2)_n-COOAlk$) on prendra soin que l'hydrolyse du groupe ester protecteur ne soit que partielle. Ainsi, par exemple, le produit de départ qu'on estérifiera avec le composé $XCR^1R^2-OCOR$ sera un copolymère d'acide $H_2N-CH\left[(CH_2)_n-COOH\right]-COOH$ et d'ester $NH_2-CH\left[(CH_2)_n-COOAlk\right]-COOH$.

Le procédé pour préparer les films de matière polymérique suivant l'invention est caractérisé par le fait qu'on dissout le polymère I ou II ou un mélange de ceux-ci dans un solvant hydrosoluble de manière à réaliser un mélange filmogène de revêtement, on applique une couche de ce mélange sur une surface à revêtir puis on met cette surface en contact avec de l'eau, celle-ci éliminant de la couche les composants hydrosolubles et formant ainsi le film de matière polymère suivant l'invention.

Comme solvants, on peut utiliser la plupart des solvants hydrosolubles énumérés ci-dessus et comme prépolymères ou oligomères solubles dans l'eau, on peut utiliser des polyalcoylène glycols tels

que, par exemple, le polyéthylène glycol ou le polypropylène glycol. On utilise habituellement le PEG 400.

On peut réaliser le film de l'invention directement sur une plaie à revêtir et à protéger. En variante, on peut utiliser une surface autre que celle d'une plaie, c'est-à-dire une surface d'un substrat quelconque inerte d'où le film, une fois formé, peut être détaché facilement. Comme telles surfaces, on peut citer des plaques de verre, de métal ou de polymères inertes tels que le polyéthylène, le polypropylène, le PVC, le plexiglass et autres. De façon générale, après avoir obtenu la solution de polymère I ou II dans un solvant convenable pour impartir au mélange une viscosité telle qu'on puisse former, avec celui-ci, des couches de revêtements, on applique celui-ci en couches minces de quelques microns à quelques mm sur la surface du substrat choisi et on trempe celui-ci dans l'eau de manière que celle-ci dissolve et entraîne les composants solubles et laisse, sur le substrat, le film protecteur de l'invention. S'il s'agit d'une plaie, le film demeure en place le temps qu'il faut, sa porosité naturelle permettant la respiration de la plaie, la diffusion progressive des humeurs et l'application, à travers la membrane, de médicaments. Par ailleurs, si la matière est faite de polymère biodégradable, celle-ci se résorbe peu à peu au cours du processus de cicatrisation et disparaît sans laisser de traces lorsque cette cicatrisation est achevée.

Si le substrat est inerte, on détache le film et on l'utilise, ultérieurement, comme pansement suivant les techniques traditionnelles. Les films de l'invention peuvent avoir, en général, de 5-10 µm à 300-500 µm d'épaisseur à l'état sec.

En variante, la matière de l'invention peut exister sous forme de membranes semi-perméables qu'on peut appliquer sur des blessures, et sous forme de mousses poreuses, recouvertes ou non d'un film semi-perméable additionnel qui sert alors de barrière contre les agents pathogènes extérieurs. On peut obtenir une telle mousse par un procédé similaire à celui déjà évoqué plus haut mais en incorporant, à la solution de polymère, des composants porogènes. Comme tels composants, on peut utiliser du sel ordinaire moulu très fin ou tout autre sel bio-compatible hydrosoluble. En effet, lors du traitement à l'eau qui suit, ce sel se dissout et laisse dans la masse

des cavités ou canaux microscopiques formant les pores de la mousse. On peut également utiliser dans la solution un solvant volatil et soumettre la couche de revêtement fraîchement appliquée (sur un substrat inerte bien entendu) à une congélation suivie d'une lyophilisation. Le solvant, s'évaporant de la couche durcie par le froid, y laisse également des cavités ou canaux constituant, ensuite, les pores de la masse.

On peut également ajouter dans la solution de polymère, lors de la préparation de la matière suivant l'invention, des substances pharmaceutiques antiseptiques comme le sulfadiazine d'argent, ou l'acétate de mafedide qui sont efficaces pour la guérison des brûlures (Journal of Traumatology, L. Zachary vol. 22(10), page 833, 1982) et, également, toutes autres sortes de médicaments antibiotiques, comme la pénicilline, ses sels et dérivés par exemple, des facteurs de croissance de tissus, etc.. c'est-à-dire, en général, tous les médicaments connus à l'usage topique pour les blessures.

Les Exemples suivant illustrent l'invention en détail.

Exemple 1

Copolymère de polyglutamate de benzoyloxyméthyle, d'acide glutamique et de glutamate de méthyle.

On a préparé de l'acide polyglutamique partiellement méthylé à partir du N-carboxyanhydride de $\gamma$-glutamate de méthyle dissous dans le chlorure de méthylène; on a utilisé la triéthylamine comme initiateur de polymérisation (A/1 = 100). On a ensuite précipité le polymère par adjonction de méthanol, puis on l'a séché sous vide. On a redissous le solide dans de l'acide trifluoracétique (TFA) de manière à réaliser une solution à 5% en poids et on a ajouté goutte à goutte en agitant vigoureusement un volume d'eau distillée suffisant pour que la solution finale contienne des volumes égaux d'eau et de TFA. On a maintenu encore 12-15 h. en agitation à température ambiante (solution visqueuse) après quoi on a versé le tout sur de l'eau distillée en grand excès, ce qui conduit à la précipitation d'un copolymère de glutamate de méthyle et d'acide polyglutamique, l'hydrolyse de l'ester méthylique ayant atteint environ 60-70%. On

a filtré ce copolymère et on l'a séché. On a controlé les proportions relatives de groupes COOH libres et estérifiées du copolymère ainsi obtenu par analyse RMN dans le TFA (intégration de la bande ester méthylique $-O-CH_3$ à 3,95 ppm). On a effectué une analyse de poids moléculaire par GPC (chromatographie par perméation de gel) dans le DMF sur colonne DUPONT ZORBAX PSM bimodale (étalonnage au polystyrène); on a mesuré un poids moléculaire moyen de Mn (number average molecular weight) de 226.000; et une dispersion (Mw/Mn) de 1,75.

On a dissous 1,22 g de copolymère dans 45 ml de DMF et on a ajouté 3,5 g de tributylamine. On a ensuite ajouté goutte à goutte 3,25 g de benzoate de chlorométhyle préparé suivant ULICH, J.A.C.S. 43, 662 (1921) et on a continué à agiter 48 heures à température ambiante. On a alors ajouté 50 ml d'eau, ce qui a causé la précipitation d'un solide incolore qu'on a filtré, séché et purifié par successivement, dissolution dans l'acétone et précipitation à l'éther, puis dissolution dans l'acétone et précipitation à l'eau. Par analyse RMN du produit dans le TFA, on a constaté la présence des résonances suivantes:

$\delta$ = 7,8 ppm (4H, aromatiques); 8,4 ppm (1H, amido); 6,5 ppm (2H; $O-CH_2-O$); 2-3 ppm (complexe, 4H, $\beta,\gamma-CH_2$) 5 ppm (1H, $\alpha-CH$); 3,95 ppm ($CH_3$ ester). L'intégration du spectre a montré que l'estérification des fonctions acide libres par le chlorométhylbenzoate était de l'ordre de 60% et que dans la formule du copolymère telle que représentée ci-dessous:

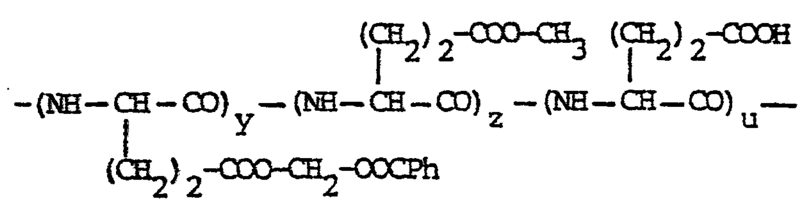

on a, pour les indices, les valeurs suivantes: y = 0,6; z = 0,33 et u = 0,07.

Ce polymère est insoluble dans l'eau, $CH_2Cl_2$, $CHCl_3$ et l'éther; il gonfle sans se dissoudre dans le méthanol et l'éthanol; il se dissout facilement dans les solvants suivants: acétone, méthyl-éthyl

cétone; THF, AcOEt/DMF, TFA, acide dichloracétique (DCA). On peut préparer des films minces de polymère en étalant sur des substrats des couches de ces solutions et en les laissant évaporer.

Exemple 2

Copolymère d'acide glutamique partiellement méthylé et de glutamate de pivaloyloxyéthyle.

Suivant le processus décrit à l'exemple précédent, on a préparé un copolymère d'acide glutamique et de glutamate de méthyle par hydrolyse ménagée du polyglutamate de méthyle dans une solution aqueuse de TFA. Le copolymère obtenu avait la formule

$$Glu(OMe)_{0,25}-Glu(OH)_{0,75}$$

On a dissous 2 g de ce copolymère dans 50 ml de DMF sec et on a ajouté goutte à goutte à cette solution 5,74 g (0,031 mole) de tributylamine et 5,09 g (0,031 mole) de pivalate d'α-chloroéthyle (préparé selon la référence citée à l'exemple précédent, par action du chlorure de pivaloyle sur le paraldéhyde). Après 4 jours d'agitation à température ordinaire, on a dilué par un excès d'eau ce qui a eu pour effet de précipiter le polymère sous forme d'une poudre incolore qu'on a redissoute, après séchage, dans de l'acétone et reprécipitée à l'éther de pétrole. On a procédé à encore deux étapes de purification par dissolution dans l'acétone et reprécipitation à l'éther de pétrole, ce qui a finalement fourni 1,8 g de produit. Par analyse RMN, on a pu établir que le copolymère répondait à la formule suivante:

$$Glu(OMe)_{0,25}-Glu(OH)_{0,6}-Glu(O-CH(CH_3)-OOCt.Bu)_{0,15}$$

Les résultats d'analyse étaient les suivants:

$\delta$ = 1,35 ppm (s, tert-butyle); $\delta$ = 1,7 - 1,8 ppm (d, $-CH(\underline{CH_3})-$)

$\delta$ = 6,8 ppm ($-\underline{CH}(CH_3)-$)

Ce polymère est soluble dans les solvants mentionnés à l'exemple précédent à l'exception du chloroforme.

Exemple 3

On a répété les opérations décrites à l'exemple précédent en utilisant, cette fois, un copolymère de formule Glu(OMe)$_{0,25}$-Glu (OH)$_{0,5}$ obtenu, comme décrit plus haut par hydrolyse ménagée du polyglutamate de méthyle, le temps de celle-ci étant limité à 6 h. On a établi que le copolymère correspondait bien à la formule susmentionnée en comparant les valeurs d'intégration des protons en RMN, le signal à 3,95 ppm ($O-CH_3$) et celui à 5 ppm (α-CH).

On a fait réagir 1 g de ce copolymère avec 2 équivalents de pivalate d' -chloroéthyle pendant 3 jours puis précipitation de sa solution dans le DMF avec de l'eau. Après purification comme décrit à l'exemple précédent, on a établi par analyse RMN, comme décrit plus haut, que la formule du copolymère s'établissait ainsi:

Glu(OMe)$_{0,5}$-Glu(OH)$_{0,33}$-Glu($OCH(CH_3)$-OCO-t.Bu)$_{0,17}$

Ce copolymère est soluble dans les solvants précités et également dans $CHCl_3$. Il est insoluble dans le polyéthylèneglycol 400.

Exemple 4

On a préparé le 3-bromophtalide de formule

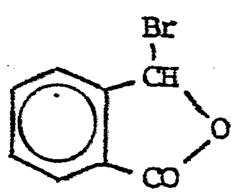

(3-bromo-1(3H)isobenzofuranne)

par bromination du phtalide d'après le document GB-A-1,364,672, page 5.

On a dissous 2 g d'acide polyglutamique et 5,74 g (2 équiv.) de tributylamine dans 35 ml de DMF sec et, à ce mélange, on a ajouté 16,7 g du bromophtalide ci-dessus. Après quelques minutes, on a constaté un épaississement important de la solution (gel) qu'on a redissous par adjonction de 5 ml d'eau. Après 6 jours d'agitation

à température ambiante, on a ajouté 500 ml d'éthanol, ce qui a provoqué la précipitation d'un produit pulvérulent incolore qu'on a filtré, essoré, rincé et séché. On a redissous le produit dans 40 ml de $CHCl_3$ et on l'a purifié en le précipitant à l'éther. Rendement 2,77 g (68%). Par analyse RMN on a obtenu les résultats suivants: $\delta$ = 8 ppm (4H, Bz + 1H, peptid.); $\delta$ = 7,7 ppm (CH lactonique); $\delta$ = 5 ppm ($\alpha$-CH); $\delta$ = 2-3 ppm (m, $-CH_2-CH_2-$).

En comparant, après intégration, le rapport entre le proton ( -CH) et les protons aromatiques on a établi que le rendement de l'estérification était de 90%. La formule du produit s'établit donc ainsi:

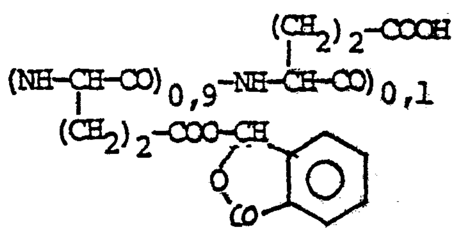

Ce produit, très soluble dans $CHCl_3$, est insoluble dans l'eau, l'alcool, le polyéthylène glycol et l'acétone pur. Il est soluble dans un mélange d'acétone $-CHCl_3$.

On a préparé un film de quelques um de ce polymère au moyen d'une solution chloroformique étalée sur une plaquette de verre et évaporée. On a placé ce film dans un récipient contenant une solution 0,1 N tampon à pH 9,5 et on a constaté une lente dégradation du polymère qu'on a observé, jour après jour, par augmentation de l'absorption UV de la solution (280 nm) due à la présence du phtalide qui se forme progressivement dans la solution tampon.

Exemple 5: Polyglutamate de pivaloyloxyméthyle

On a préparé de l'acide polyglutamique à partir du N-carboxyanhydride de $\gamma$-glutamate de méthyle dissous dans le chlorure de méthylène; on a utilisé la triéthylamine comme initiateur de polymérisation (A/I=100). On a ensuite précipité le polymère par adjonction de méthanol, puis on l'a séché sous vide. On a redissous le solide dans de l'acide trifluoracétique (TFA) de manière à réaliser une solution à 5% en poids et on a ajouté goutte à goutte en agitant vigoureusement un volume d'eau distillée suffisant pour que la solution finale contienne des volumes égaux d'eau et de TFA. On a maintenu encore 24 h. en agitation à température ambiante (solution visqueuse) après quoi on a versé le tout sur de l'eau distillée en grand excès, ce qui conduit à la précipitation de l'acide polyglutamique. On a filtré cet acide et on l'a séché. On a controlé la pureté de l'acide ainsi obtenu par analyse RMN dans le TFA (absence de la bande ester méthylique $-O-CH_3$ à 4,5 ppm). On a effectué une analyse de poids moléculaire par GPC (chromatographie par perméation de gel) dans le DMF sur colonne DUPONT ZORBAX PSM bimodale (étalonnage au polystyrène) et on a mesuré les valeurs suivantes: $M_n$ = 226.000; $M_w$ = 397.000; $M_z$ = 723.000; dispersivité = 1,75.

On a dissous le polyacide dans le DMF à raison de 5% en poids et, à 50 ml de cette solution, on a ajouté 4 ml d'eau et 4,04 g (0,04 mole) de triéthylamine. On observe d'abord une précipitation du polyglutamate de triéthylamine, ce sel se redissolvant ensuite par agitation lorsqu'on ajoute l'eau. Lorsque tout a été dissous, on a ajouté goutte à goutte 6,02 g de pivalate de chlorométhyle (Fluka AG) et on a continué à agiter 48 heures à température ambiante. On a alors ajouté 50 ml d'eau, ce qui a causé la précipitation d'un solide incolore qu'on a filtré, séché et purifié par successivement, dissolution dans l'acétone et précipitation à l'éther, puis dissolution dans l'acétone et précipitation à l'eau. Par analyse RMN du produit dans le TFA, on a constaté la présence d'un pic tertbutyle à 1,35 ppm. L'intégration du spectre a montré que l'estérification de l'acide polyglutamique par le chlorométhylpivalate était totale.

Le poids moléculaire a été déterminé comme ci-dessus et a fourni les résultats suivants: $M_n$ = 30.480; $M_w$ = 708.000; $M_z$ = 297.000; dispersivité = 3,54 (viscosité relative $\eta$ = 1,57, C = 0,2 g/dl dans le DMF). Rendement final 51%.

Ce polymère est insoluble dans l'eau, $ClCH_2CH_2Cl$, $CHCl_3$ et l'éther; il gonfle sans se dissoudre dans le méthanol et l'éthanol; il se dissout facilement dans les solvants suivants: acétone, méthyl-éthyl cétone; THF, AcOEt/DMF, TFA, acide dichloracétique (DCA). On peut préparer des films minces de polymère en étalant sur des substrats des couches de ces solutions et en les laissant évaporer. Le spectre IR confirme également la structure proposée (forte bande à 1740 $cm^{-1}$).

Exemple 6

On a préparé un film mince du polymère obtenu suivant l'exemple 5 en étalant une couche de solution à 10% dans l'acétone sur une plaque de verre et en l'immergeant, quelques minutes dans un grand excès d'eau à température ordinaire.

On a ensuite immergé plusieurs échantillons de ce film dans une solution à 1% de pancréatine dans du tampon phosphate 0,1 M (pH 7,5) et on a abandonné ces échantillons à température ambiante pendant 1 à 8 jours. On a constaté, après 1 jour, une perte notable de résistance mécanique (env. 50%) et après 2 jours, une disparition quasi totale de cette résistance (l'échantillon s'est fragmenté en morceaux). Après 8 jours, l'échantillon s'était entièrement dégradé et n'était plus visible. Des résultats analogues ont été observés en utilisant de l'estérase ou de la leucinaminopeptidase.

Exemple 7

Préparation d'un copolymère ester pivaloyloxyméthyl-glutamique et leucine.

On a opéré comme dans l'exemple 6, mais en partant d'un copolymère 85/15 d'acide polyglutamique et de leucine et on a obtenu un copolymère 85/15 de pivaloyloxyméthyl glutamate/leucine. Le spectre RMN du polymère en solution dans le TFA montre que 100% des groupes carboxyliques latéraux de l'acide polyglutamique sont estérifiés par le chlorométhylpivalate. Le polymère est également soluble dans l'acétone, le MEK, la DMF, le THF. Il gonfle dans les alcools, sans se dissoudre. Spectre RMN: $\delta$ = 1,35 ppm 9 protons tert-butyle; $\delta$ = 1,0,6 protons isobutyle. Intégration: rapport des groupements 85:15.

Exemple 8

Préparation d'un polymère ester d'isobutyloxyméthyl glutamate.

On a effectué la synthèse en partant d'acide polyglutamique préparé comme décrit dans l'exemple 5; on a dissous ce polymère dans du DMF préalablement séché sur tamis moléculaire de manière à obtenir une solution à 3,2%. A 20 g de cette solution, on a ajouté 1,83 g de tributylamine (2 équivalents). Contrairement à ce qui a été observé avec la triéthylamine, il n'est pas nécessaire d'ajouter de l'eau pour que la dissolution s'effectue. On ajoute ensuite goutte à goutte 1,36 g d'ester chlorométhylique de l'acide isobutyrique (préparé suivant ULICH, J.A.C.S. 43, 662, (1921)) (Eb. 65°C/48 Torr) à la solution de polyglutamate de tributylamine et on laisse 3 jours sous agitation à température ambiante. Le mélange réactionnel est ensuite précipité par l'eau, redissous dans l'acétone, reprécipité par l'exane, redissous dans l'acétone et précipité à nouveau dans l'eau. L'analyse RMN du polymère séché montre la présence de pics isobutyle ($\delta$ = 1,15 ppm, doublet) caractéristiques du produit recherché et, par intégration, 90% de substitution (obtenu 1,03 g, 90%). Le polymère est également soluble dans l'acétone, la MEK, le THF, le DMF. Le spectre infrarouge d'un film coulé à partir d'une solution de TFA, ainsi que l'analyse élémentaire correspondent à la structure proposée.

## Exemple 9

Préparation pharmaceutique à base de polyglutamate de pivaloyloxyméthyle plastifié au PEG:

On dissout 5 g de poly-pivaloyloxyméthylglutamate et 5 g de polyéthylèneglycol 600 (Fluka, AG) et 2 g de diazepam (Hoffman-LaRoche) dans 100 ml d'acétone. On coule un film à partir de cette solution sur une plaque de verre et on la traite à l'eau jusqu'à dissolution du PEG. Le film obtenu, chauffé à 50°C, fond et peut être moulé sous forme de capsules qui, une fois refroidies et plongées dans une solution aqueuse isotonique, laissent diffuser du diazepam dans le milieu.

## Exemple 10

Synthèse de poly-acétoxyméthylglutamate.

On a effectué la synthèse comme dans l'exemple 8 mais en partant de chlorométhylacétate.

Le polymère obtenu est également soluble dans l'acétone. Son spectre RMN montre la présence d'un pic à 3,2 ppm, caractéristique du groupe acétyle (3 protons). Le polymère se dissout dans la soude caustique aqueuse N/100 en s'hydrolysant en quelques minutes.

Exemple 11

Biodégradation in vitro du poly-pivaloyloxyméthylglutamate.

On a préparé, suivant la méthode de l'exemple 5, du poly-pivaloyloxyméthylglutamate dont le groupement méthyle est marqué au $^{14}C$. On a pour cela synthétisé du chlorométhylpivalate en faisant réagir du chlorure de pivaloyle sur du paraformaldéhyde marqué au $^{14}C$, selon la technique décrite dans J.A.C.S., 89 (21), 5442, (1967).

L'activité spécifique du polymère, mesurée par combustion est de 3 µCie/g. On a préparé avec ce polymère des films de 3 x 3 cm à partir de solutions dans l'acétone ou dans le TFA et en traitant ensuite des couches de revêtements à l'eau. Les films obtenus sont trempés soit dans des solutions enzymatiques de Leucineaminopeptidase de rein de porc (Sigma, 3,7 Unités/ml, tampon 0,1 tris, 5 mM $MgCl_2$, pH 8,4), soit d'estérase de foie de porc, (Sigma, 11,6 Unités/ml, tampon 0,1M Tris, pH 7,5). On mesure la vitesse de dégradation en observant, d'une part, l'aspect du polymère et, d'autre part, en comptant le degré de radioactivité acquise par la solution. On a renouvelé les solutions enzymatiques chaque jour. On a obtenu les résultats suivants:

| temps d'incubation | Esterase (pH 7,5) | (pH 8,4) Leucine Aminopeptidase | |
|---|---|---|---|
| film coulé à partir d'une solution de: | Acétone | Acétone | TFA |
| 1 jour | film opaque, 3,5 % hydrolyse | film opaque, 13,6 % hydrolyse | film opaque, . 12,6 % hydrolyse |
| 2 jours | film blanchâtre, soufflé, 9 % hydrolyse | film opaque, 29 % hydrolyse | film très déformé, 46,8 % hydrolyse |
| 4 jours | film blanchâtre, soufflé, 17 % hydrolyse | film opaque, soufflé, 52 % hydrolyse | film désagrégé, dissous, quelques débris solides, 85 % hydrolyse |
| 5 jours | film gonflé, 24 % hydrolyse | film commence à se casser, 71 % hydrolyse | |
| 7 jours | film gelatineux. 37 % hydrolyse | film entièrement désagrégé, 85 % hydrolyse | |

Exemple 12

On a préparé un copolymère d'acide aspartique et de leucine (voir Polymer 16, 735 (1975) en copolymérisant du β-benzylaspartate NCA et de la leucine NCA (N-carboxyanhydrides) en proportions équimoléculaires. On transestérifie les groupes benzyl ester par le méthanol pour obtenir le copolymère poly-(β-méthylaspartate/leucine). On saponifie ensuite les groupes méthylester par la soude N/10 dans le méthanol pour obtenir l'acide poly-[Asp(OH)/leu]. L'analyse des acides aminés montre qu'on a une proportion de leucine de 56% et de 44% pour l'acide aspartique. Le poids moléculaire $M_n$ est de 35.000 (mesuré par GPC).

On dissout 1 g de poly-[Asp(OH)/leu] dans 20 g de DMF sec. On ajoute 3,25 g de tributylamine (4 équivalents par rapport aux restes Asp(OH)) et 2,64 g de chlorométhylpivalate (4 équivalents). On laisse sous agitation 3 jours à température ambiante. On précipite le polymère dans $H_2O$ distillée, on le redissout dans l'acétone, on précipite dans l'éther de pétrole, on redissout dans l'acétone, on reprécipite avec $H_2O$. On sèche.

L'analyse RMN montre qu'on a bien obtenu du poly-(pivaloyloxyméthylaspartate/leucine) le taux d'estérification étant d'environ 20% ($\delta$ = 7,5 ppm protons benzyliques; $\delta$ = 8 ppm, protons NH; $\delta$ = 5,4 et 4,8 protons alpha-CH; $\delta$ = 3,95, protons $-O-CH_3$ non saponifiés; $\delta$ = 3,3 et 2, protons $-CH_2-$, $\delta$ = 1,35 ppm, protons t- butyle). Malgré le taux d'estérification relativement faible, le copolymère est soluble dans l'acétone.

Exemple 13

A titre comparatif, on a étudié les vitesses d'hydrolyse en milieu alcalin d'un copolymère suivant l'invention marqué comme décrit à l'exemple 7, (copolymère glutamate de pivaloyloxyméthyl-leucine 85-15 suivant l'exemple 8) et d'un copolymère de polyglutamate de méthyle-leucine 85-15, de l'art antérieur préparé par les techniques connues habituelles au moyen de méthanol marqué au $^{14}C$. Le polymère suivant l'invention (A) a été converti en un film d'environ 0,25 mm par dépôt sur une plaque de verre d'une couche de solution acé-

tonique qu'on a ensuite soumise à l'eau comme auparavant. Pour le film de contrôle (B) on a procédé de même à partir d'une solution dans l'acide TFA. On a soigneusement lavé ce dernier film à l'eau afin d'éliminer toute trace de TFA.

On a plongé les films (A) et (B) dans des solutions aqueuses à pH 9,5 (tampon phosphate) en lente agitation et, à intervalles, on a mesuré la radioactivité des produits dissous dans ces solutions d'où on a calculé les taux d'hydrolyse des polymères soumis au test.

On a trouvé les résultats suivants exprimés en % en poids de matière hydrolysée après un temps donné (heures).

| Echantillon (A) | | Echantillon (B) | |
|---|---|---|---|
| temps (h) | % en poids | temps (h) | % en poids |
| 1,5 | 2,8 | 18 | 1,8 |
| 2,5 | 5,0 | 42 | 4,2 |
| 4,5 | 13,2 | 64 | 5 |
| 8 | 37,25 | 88 | 5,9 |
| 10 | dissolution | 112 | 6,8 |

On voit, d'après les résultats ci-dessus que le copolymère (A) se dégrade à tel point en 8-10 h que le médicament qu'il aurait pu contenir se serait dissous entièrement dans de telles conditions, alors que le copolymère de l'art antérieur n'est que très peu altéré après 112 h dans les mêmes conditions.

En faisant varier le taux de leucine du copolymère (A), on peut faire varier le temps d'hydrolyse, dans le sens d'une augmentation en augmentant ledit taux et d'une diminution en réduisant ledit taux.

Exemple 14

On dissout 3 g du polymère préparé suivant l'exemple 5 dans 150 ml d'acétone et on ajoute 30 g de PEG 400 (Pharmacopéia Helvetica). Après évaporation de cette solution au rotavapor jusqu'à élimination totale de l'acétone, sous vide, on obtient une pâte visqueuse transparente d'une viscosité de 300 poises environ.

On étale à l'aide d'une spatule la pâte préparée comme ci-dessus sur une plaie pratiquée par ablation d'un morceau de peau sur un rat d'expérience de manière à former une couche mince et uniforme (50 à 100 µm). Puis, on arrose la zone à l'aide d'eau distillée stérile au moyen d'une pissette. Il se forme immédiatement une mince pellicule transparente, on continue à arroser pendant environ 2 minutes pour éliminer le PEG 400. Le film résiduel adhère bien à la plaie mais peut cependant être enlevé, si nécessaire, à l'aide de pincettes. Il est souple et épouse parfaitement toutes les formes de la partie où il est appliqué. Il contient encore des traces de PEG non-lixiviable.

Après le lavage à l'eau pendant 2 minutes, le film de polymère obtenu a une épaisseur de 30 à 40 um environ. On a mesuré la perméabilité à l'eau liquide de ce film en maintenant d'un côté du film de l'eau liquide et en mesurant de l'autre côté le passage d'eau sous forme de vapeur, en la piégeant dans du $P_2O_5$ et en mesurant l'augmentation de poids du $P_2O_5$ en fonction du temps. On a trouvé une perméabilité de 5 $mg/h/cm^2$. Chvapil (J. Biomed. Mater. Research 16, 245 (1982)) recommande une perméabilité optimale de 2,5 à 6 $mg/h/cm^2$ pour des pansements.

Exemple 15

On prépare la pâte comme dans l'exemple 1 mais on ajoute 1% en poids du polymère de sulfadiazine d'argent en suspension à la composition de revêtement. Puis on dépose un revêtement de la composition comme à l'exemple précédent et on trempe la zone revêtue dans l'eau. Il se forme un film adhérent qui, peu à peu, relargue le médicament et favorise la guérison de la plaie.

Exemple 16

Préparation d'une membrane semi-perméable.

On prépare une solution de 0,5 g du polymère de l'exemple 5 dans 6,5 g d'acétone et on ajoute 3,3 g de formamide. On applique une couche de cette solution sous forme de film mince (500 µm) à l'aide d'une raclette sur une plaque de verre. On laisse évaporer la solution d'acétone pendant 1 minute à température ambiante puis on trempe le gel obtenu dans une solution d'eau à 0°C. Après 1 heure, la membrane solide s'est formée et on la rince avec de l'eau distillée pendant 2 heures. La membrane obtenue est blanche et a une perméabilité élevée à l'eau liquide ou vapeur ( > 10 mg/h/cm$^2$) mais interdit le passage de bactéries. Elle peut être appliquée sur des blessures et maintenue sur place à l'aide d'un bandage.

Exemple 17

On prépare une solution du poly-[POMEG/leucine] préparé selon l'exemple 7 par dissolution de 2 g de ce polymère dans 20 ml de diméthylformamide. On ajoute à la solution 2 g de NaCl (puriss) pilé finement et on mélange. La pâte obtenue est étalée à l'aide d'une racle sur une plaque de téflon et on évapore le DMF sous vide (1 mmHg, 40°C, 24 heures). La feuille obtenue est ensuite trempée dans une solution d'eau distillée à laquelle on ajoute un peu d'HCl de manière à maintenir le pH vers 3-4. On agite pendant 24 heures de manière à dissoudre le reste du DMF et le sel jusqu'à obtention d'une mousse poreuse d'épaisseur 1 mm environ et comportant des pores de 10 à 100 µm. Cette mousse est souple et peut être appliquée sur des blessures et maintenue par un bandage.

Exemple 18

Lyophilisation d'une solution dioxanne.

On mélange du polymère comme celui des exemples 16 ou 17 dans du dioxanne de manière à avoir une solution visqueuse à 15% de polymère; on coule cette solution sur une plaque de verre silanisée (pour éviter l'adhésion) et on la refroidit à -15°C. On lyophilise ensuite cette plaque en maintenant un léger chauffage sous la plaque, la mousse obtenue est très poreuse et ressemble à de la mousse de polystyrène. Avec une épaisseur de 1 mm, elle est assez rigide mais peut être obtenue beaucoup plus souple en rajoutant avant la lyophilisation 10% (du poids du polymère) de PEG 400, qui reste après lyophilisation. Cette mousse peut être utilisée pour couvrir les blessures et peut être avantageusement recouverte d'un film se-

mi-perméable en surface pour contrôler les vitesses d'évaporation (pansement en 2 couches).

## R E V E N D I C A T I O N S

pour les états contractants: BE,CH,DE,FR,GB,IT,LU,NL,SE

1. Matière polymérique filmogène utilisable comme pansement, caractérisée par le fait qu'elle consiste en un polypeptide, en solution dans un solvant hydrosoluble, constitué de polyaspartate ou polyglutamate d'acyloxyméthyle ou d'aroyloxyméthyle biodégradable de formule

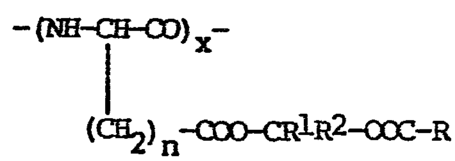

(I)

dans laquelle $R^1$ et $R^2$ sont des alcoyles ou l'hydrogène, R étant un reste aliphatique ou aromatique substitué ou non; ou bien $R^2$ est un alcoyle ou l'hydrogène et R et $R^1$ sont liés l'un à l'autre sous forme d'un pont éthylène ou vinylène substitué ou non; et ses copolymères avec d'autres acides aminés de formule

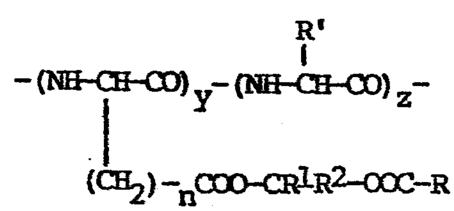

(II)

où les groupes R, $R^1$ et $R^2$ ont le sens donné ci-dessus et où R' est un reste d'amino-acide libre ou partiellement ou totalement estérifié; n vaut 1 ou 2 et x qui est égal à y + z est choisi pour que la masse moléculaire du polypeptide ne soit pas inférieure à 5000 D.

2. Matière polymérique filmogène suivant la revendication 1, caractérisée par le fait que R est choisi parmi les restes méthyle, éthyle, isopropyle, isobutyle, tert-butyle, phényle et benzyle.

3. Matière polymérique filmogène suivant la revendication 1, caractérisée par le fait que, R et $R^1$ étant liés l'un à l'autre, le maillon formé par ceux-ci est choisi parmi les formules suivantes: $-CH_2-CH_2-$; $-CH=CH-$; $-CH(CH_3)-CH(CH_3)$; $-C(CH_3)=C(CH_3)-$; 1,2-phénylène; cyclohexénylène; cyclopenténylène, cyclopentadiénylène.

4. Matière polymérique filmogène suivant la revendication 1, caractérisée par le fait que le composé I est choisi parmi les glutamate et aspartate d'acyl- et aroyl-oxyméthyle et que le composé II est choisi parmi les copolymères de glutamate ou aspartate d'acyl- et d'aroyl-oxyméthyle avec un ou plusieurs autres acides aminés choisis parmi l'alanine, la leucine, la valine et la phénylalanine.

5. Matière polymérique filmogène suivant la revendication 1, caractérisée par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

6. Matière polymérique filmogène suivant la revendication 4, caractérisée par le fait que les groupes acyles sont choisis parmi les groupes acétyle, propionyle, butyryle, pivaloyle, benzoyle et phénylacétyle.

7. Matière polymérique filmogène suivant la revendication 1, caractérisée par le fait que le solvant est un polyalcoylène glycol, par exemple le polyéthylène glycol (PEG).

8. Matière filmogène suivant la revendication 7, caractérisée par le fait que le solvant est au moins l'un des solvants suivants: PEG, diméthylformamide (DMF), acétone, méthyléthylcétone (MEK), butanol, éthanol, dioxanne, tetrahydrofuranne (THF).

9. Matière polymérique filmogène suivant la revendication 1, caractérisée par le fait que ladite solution contient un agent porogène.

10. Utilisation de la matière polymérique filmogène suivant les revendications précédentes pour appliquer un film polymère sur un substrat, caractérisée par le fait qu'on applique celle-ci sous forme d'une couche à la surface de ce substrat puis qu'on élimine le solvant que contient cette couche.

11. Utilisation de la matière polymérique filmogène suivant la revendication 10, caractérisée par le fait qu'on applique une couche de la solution sur une surface à revêtir, puis on traite cette surface ainsi revêtue par de l'eau, celle-ci ayant pour effet de dissoudre et d'éliminer le solvant hydrosoluble de manière à former le film de polymère.

12. Utilisation suivant la revendication 11, caractérisée par le fait que ledit agent porogène étant le sel moulu très fin, la dissolution des grains de celui-ci dans l'eau de traitement donne lieu à la formation d'une structure de mousse poreuse.

13. Utilisation suivant la revendication 10, caractérisée par le fait que ledit solvant étant un solvant organique volatil, on soumet la couche de revêtement à une lyophilisation de manière que, par évaporation du solvant volatil, il se forme dans la masse une structure poreuse.

## REVENDICATIONS
## pour l'état contractant: AT

1. Procédé de préparation d'une solution de matière polymérique filmogène utilisable comme pansement, caractérisé par le fait qu'on dissout dans un solvant hydrosoluble un polypeptide constitué de polyaspartate ou polyglutamate d'acyloxyméthyle ou d'aroyloxyméthyle biodégradable de formule

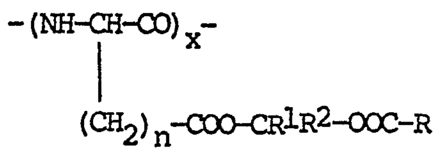

(I)

dans laquelle $R^1$ et $R^2$ sont des alcoyles ou l'hydrogène, R étant un reste aliphatique ou aromatique substitué ou non; ou bien $R^2$ est un alcoyle ou l'hydrogène et R et $R^1$ sont liés l'un à l'autre sous forme d'un pont éthylène ou vinylène substitué ou non; ou ses copolymères avec d'autres acides aminés de formule

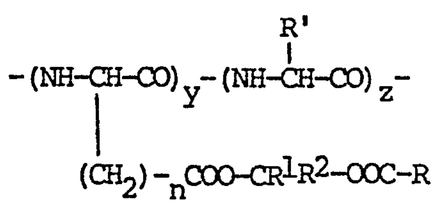

(II)

où les groupes R, $R^1$ et $R^2$ ont le sens donné ci-dessus et où R' est un reste d'amino-acide libre ou partiellement ou totalement estérifié; n vaut 1 ou 2 et x qui est égal à y + z est choisi pour que la masse moléculaire du polypeptide ne soit pas inférieure à 5000 D.

2. Procédé suivant la revendication 1, caractérisé par le fait que R est choisi parmi les restes méthyle, éthyle, isopropyle, isobutyle, tert-butyle, phényle et benzyle.

3. Procédé suivant la revendication 1, caractérisé par le fait que, R et $R^1$ étant liés l'un à l'autre, le maillon formé par ceux-ci est choisi parmi les formules suivantes: $-CH_2-CH_2-$; $-CH=CH-$;

$-CH(CH_3)-CH(CH_3)$; $-C(CH_3)=C(CH_3)-$; 1,2-phénylène; cyclohexénylène; cyclopenténylène; cyclopentadiénylène.

4. Procédé suivant la revendication 1, caractérisé par le fait que le composé I est choisi parmi les glutamate et aspartate d'acyl- et aroyl-oxyméthyle et que le composé II est choisi parmi les copolymères de glutamate ou aspartate d'acyl- et d'aroyl-oxyméthyle avec un ou plusieurs autres acides aminés choisis parmi l'alanine, la leucine, la valine et la phénylalanine.

5. Procédé suivant la revendication 1, caractérisé par le fait que le copolymère II est choisi parmi les copolymères des polyglutamate ou aspartate I avec, respectivement, l'acide glutamique et/ou les glutamates d'alcoyles inférieurs et l'acide aspartique et/ou les aspartates d'alcoyles inférieurs.

6. Procédé suivant la revendication 4, caractérisé par le fait que les groupes acyles sont choisis parmi les groupes acétyle, propionyle, butyryle, pivaloyle, benzoyle et phénylacétyle.

7. Procédé suivant la revendication 1, caractérisé par le fait que le solvant est un polyalcoylène glycol, par exemple le polyéthylène glycol (PEG).

8. Procédé suivant la revendication 7, caractérisé par le fait que le solvant est au moins l'un des solvants suivants: PEG, diméthylformamide (DMF), acétone, méthyléthylcétone (MEK), butanol, éthanol, dioxanne, tetrahydrofuranne (THF).

9. Procédé suivant la revendication 1, caractérisé par le fait que ladite solution contient un agent porogène.

10. Utilisation de la solution préparée suivant les revendications précédentes pour appliquer un film polymère sur un substrat, caractérisée par le fait qu'on applique celle-ci sous forme d'une couche à la surface de ce substrat puis qu'on élimine le solvant que contient cette couche.

11. Utilisation suivant la revendication 10, caractérisée par le fait qu'on applique une couche de la solution sur une surface à revêtir, puis on traite cette surface ainsi revêtue par de l'eau, celle-ci ayant pour effet de dissoudre et d'éliminer le solvant hydrosoluble de manière à former le film de polymère.

12. Utilisation suivant la revendication 11, caractérisée par le fait que ledit agent porogène étant le sel moulu très fin, la dissolution des grains de celui-ci dans l'eau de traitement donne lieu à la formation d'une structure de mousse poreuse.

13. Utilisation suivant la revendication 10, caractérisée par le fait que ledit solvant étant un solvant organique volatil, on soumet la couche de revêtement à une lyophilisation de manière que, par évaporation du solvant volatil, il se forme dans la masse une structure poreuse.

Office européen des brevets

# RAPPORT DE RECHERCHE EUROPEENNE

0159293
Numéro de la demande

EP 85 81 0146

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int Cl 4) |
|---|---|---|---|
| A | FR-A-2 150 695 (AJINMOTO)<br>--- | | A 61 L 15/06<br>C 08 G 69/10 |
| A | FR-A-2 503 561 (SETON)<br>--- | | |
| A | DE-A-2 849 570 (MAX-PLANCK-GESELLSCHAFT ZUR FÖRDERUNG DER WISSENSCHAFTEN)<br>----- | | |
| | | | DOMAINES TECHNIQUES RECHERCHES (Int. Cl.4) |
| | | | A 61 L<br>C 08 G |

Le présent rapport de recherche a été établi pour toutes les revendications

| Lieu de la recherche | Date d'achèvement de la recherche | Examinateur |
|---|---|---|
| LA HAYE | 03-07-1985 | LEROY ALAIN |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82